# EUROPÄISCHE PATENTANMELDUNG

(11) **EP 0 075 141 A1**
(43) Veröffentlichungstag der Anmeldung: **30.03.1983**
(21) Anmeldenummer: 82107954.8
(22) Anmeldetag: 30.08.1982
(51) Int. Cl.: C07D 271/08, A61K 31/41

(54) **1,2,5-Oxadiazol-3,4-dicarbonsäurederivate, Verfahren zu ihrer Herstellung, ihre Verwendung und sie enthaltende pharmazeutische Zubereitungen**

(30) Priorität: 03.09.1981 DE 3134849
(71) Anmelder: CASSELLA Aktiengesellschaft, D-60386 Frankfurt (DE)
(72) Erfinder: Schönafinger, Karl, Dr., D-8531 Uehlfeld (DE); Beyerle, Rudi, Dr., D-6000 Frankfurt am Main 60 (DE); Martorana, Piero A., Dr., D-6380 Bad Homburg (DE); Nitz, Rolf-Eberhard, Dr., D-6000 Frankfurt am Main 60 (DE)
(74) Vertreter: Urbach, Hans-Georg, Dr.

(57) **Zusammenfassung**

1,2,5-Oxadiazol-3,4-dicarbonsäurederivate der Formel 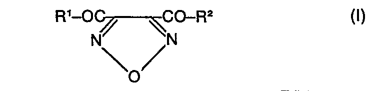 worin
R' die Gruppe -OH, -OR', -NH₂, -NHR⁴ oder -NR⁵R⁶,
R² die Gruppe -NHR⁴ oder -NR⁵R⁶,
R³ eine Alkylgruppe mit 1 bis 4 C-Atomen,
R⁴ eine Alkylgruppe mit 1 bis 6 C-Atomen, eine Cycloalkylgruppe mit 5 oder 6 C-Atomen, eine Aralkylgruppe, die gegebenenfalls mit 1 oder 2 Methoxygruppen substituiert sein kann, oder eine Heteroarylalkylgruppe, eine -CH₂-CH₂(CH₂)ₘ-OCH₃-Gruppe, worin m 0 oder 1 bedeutet, eine 2-Hydroxyethylgruppe oder eine basisch substituierte Alkylgruppe bedeuten und
R⁵ und R⁶ unabhängig voneinander eine der Bedeutungen von R⁴ besitzen oder zusammen mit dem Stickstoffatom, an das sie gebunden sind, einen gegebenenfalls substituierten heterocyclischen Ring bilden, werden dadurch hergestellt, daß ein 1,2,5-Oxadiazol-3,4-bis-carbonsäureester der Formel II 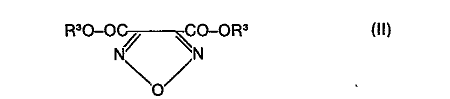 mit mindestens 2 Molen eines Amins R⁴NH₂ oder R⁵R⁶NH umgesetzt wird, oder mit weniger als 2 Molen, insbesondere 1 bis 1,1 Molen, eines Amins R⁴NH₂ bzw. R⁵R⁶NH umgesetzt wird und gegebenenfalls anschließend mit NH₃ oder einem anderen Amin R⁴NH₂ oder R⁵R⁶NH umgesetzt oder zur Abspaltung der R³O-Gruppe hydrolysiert wird oder daß aus einem 1,2,5-Oxadiazol-2-oxid-3,4-bis(carbonsäureamid) der Formel III 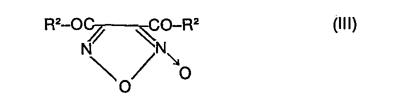 der Oxidsauerstoff durch Reduktion entfernt wird.

Sie werden als pharmakologische Wirkstoffe verwendet.

## Beschreibung

Die vorliegende Erfindung betrifft 1,2,5-Oxadiazol-3,4-dicarbonsäurederivate der allgemeinen Formel I 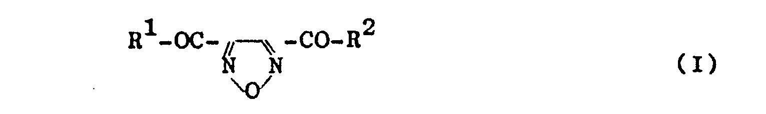 und ihre pharmakologisch annehmbaren Säureadditionsverbindungen, Verfahren zu ihrer Herstellung, ihre Verwendung und sie enthaltende pharmazeutische Zubereitungen.

In der Formel I bedeuten:
R¹ die Gruppe -OH, -OR³, -NH₂, -NHR⁴ oder -NR⁵R⁶,
R² die Gruppe -NHR⁴ oder -NR⁵R⁶,
R³ eine Alkylgruppe mit 1 bis 4 C-Atomen,
R⁴ eine Alkylgruppe mit 1 bis 6 C-Atomen, eine Cycloalkylgruppe mit 5 oder 6 C-Atomen, eine Aralkylgruppe, die gegebenenfalls mit 1 oder 2 Methoxygruppen substituiert sein kann, oder eine Heteroarylalkylgruppe, eine -CH₂-CH₂-(CH₂)ₘOCH₃-Gruppe, worin m O oder 1 bedeutet, eine 2-Hydroxyethylgruppe oder eine basisch substituierte Alkylgruppe und
R⁵ und R haben unabhängig voneinander eine der Bedeutungen von R⁴ oder bilden zusammen mit dem Stickstoffatom, an das sie gebunden sind, einen heterocyclischen Ring, der gegebenenfalls auch substituiert sein kann.

Die für R³,R⁴,R⁵ und R stehenden Alkylgruppen können geradkettig oder verzweigt sein. Für R ³ ist der Ethylrest bevorzugt.Als für R⁴ stehende Alkylreste sind solche mit 1 bis 4 C-Atomen bevorzugt. Als Cycloalkylgruppen für R⁴ sind Cyclopentyl und insbesondere Cyclohexyl zu nennen. Für die für R⁴ stehende Aralkylgruppe sind die Benzyl- und Phenethylgruppe bevorzugt, die beide in ihren Phenylkernen auch durch eine oder zwei Methoxigruppen substituiert sein können. Als tür R stehende Heteroarylalkylgruppen kommen zum Beispiel Pyridyl-ethyl-, insbesondere Pyridyl-methyl-gruppen in Betracht. Für R⁴ stehende, basisch substituierte Alkylgruppen besitzen z.B. den Rest R⁷R⁸N-CH₂-(CH₂)ₙ-CH₂-, worin R⁷ und R⁸ unabhängig voneinander geradkettige oder verzweigte Alkylreste mit 1 bis 4 C-Atomen, vorzugsweise Methyl oder Ethyl, und n O oder vorzugsweise 1 bedeuten. Die Reste R⁷ und R⁸ können auch zusammen mit dem Stickstoffatom, an das sie gebunden sind, einen, gegebenenfalls substituierten, heterocyclischen Rest bilden.

Die für R⁵ und R⁶ stehenden Alkylreste besitzen insbesondere 1 bis 4 C-Atome und bedeuten vorzugsweise Methyl oder Ethyl. Die Reste R5 und R⁶ können auch, ebenso wie die Reste R⁷ und R , zusammen mit dem Stickstoffatom, an das sie gebunden sind, einen gegebenenfalls substituierten heterocyclischen Ring bilden. Diese heterocyclischen Ringe sind vorzugsweise gesättigt und besitzen vorzugsweise 5 oder 6 Glieder. Neben dem einen Stickstoffatom, über das sie an das restliche Molekül gebunden sind, können sie noch 1 bis 2 weitere Heteroatome, vor allem Stickstoff, Sauerstoff oder Schwefel,enthalten. Vorzugsweise enthalten die heterocyclischen Ringe ein oder zwei N-Atome oder ein N- und ein O- oder S-Atom. Beispielsweise können die Reste R⁵ und R bzw. Rund R⁸ zusammen mit dem Stickstoffatom, an das sie gebunden sind, einen Morpholino-, einen 1-Piperazinyl-, einen Piperidino- oder I-Pyrrolidinyl-Rest bedeuten. Die heterocyclischen Reste können z.B. durch Alkyl mit 1 bis 4 C-Atomen, insbesondere Methyl oder Ethyl, durch Phenyl, Tolyl, Methoxiphenyl, Dimethoxiphenyl substituiert sein.

In den Verbindungen der Formel I, die sowohl in R - als auch in R²-Position einen -NHR⁴-Rest besitzen, kann die Bedeutung von R⁴ für beide R⁴ -Reste gleich oder verschieden sein. Ebenso kann in Verbindungen der Formel I, die sowohl in R - als auch in R²-Position einen -NR⁵R⁶-Rest besitzen, die Bedeutung für beide R⁵ und beide R⁶ Reste gleich oder verschieden sein. Verbindungen der Formel I, die in den Resten R¹ und R² ᵢden- tische Substitutenteh besitzen, sind bevorzugt.

Die neuen Verbindungen der Formel I, die in R¹- und R -Stellung identisch substituiert sind und die den Formeln Ia bzw. Ib entsprechen, 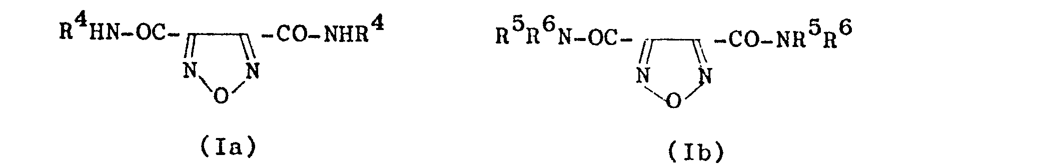 lassen sich in einfacher Weise dadurch herstellen, daß man einen 1,2,5-Oxadiazol-3,4-bis-carbonsäureester der Formel II 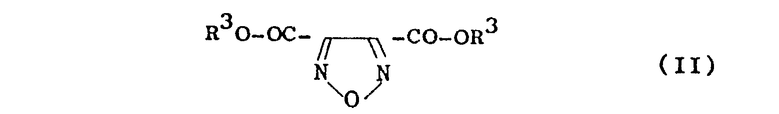 worin R³ vorzugsweise Ethyl bedeutet, mit mindestens 2 mol des Amins R NH2 bzw. R⁵R⁶NH umsetzt. In der Regel brauchen nicht mehr als 2,5 mol des Amins eingesetzt zu werden.

Diese Umsetzung kann durch Vereinigen der Komponenten vorgenommen werden. Normalerweise ist es jedoch zweckmäßig, eine Ausgangskomponente, vorzugsweise aber beide Ausgangskomponenten, in einem geeigneten inerten Lösungs- oder Dispergiermittel zu lösen oder zu dispergieren und die Komponenten in dieser Form langsam, z.B. durch Zutropfen, zu vereinigen. Die Reaktionstemperaturen betragen je nach Reaktivität des verwendeten Amins -10°C bis 120°C, insbesondere 0°C bis 120°C. In vielen Fällen sind Temperaturen von 0 bis 30° ausreichend. Geeignete Lösungs- oder Dispergiermittel sind z.B. Alkohole, insbesondere solche mit 1 bis 6 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methyl-ethyl-ether, Di-n-propyl-ether, Di-isopropyl-ether, Methyl-n-butyl-ether, Ethylpropyl-ether, Di-butyl-ether, Tetrahydrofuran, 1,4-Dioxan, 1,2-Dimethoxyethan, Bis-ß-methoxyethylether; Polyether, wie z.B. Polyethylenglykole mit einem Molekulargewicht bis ca. 600; Oligoethylen-glycol-dimethyl-ether, wie z.B. Pentaglyme; Kronenether, d.h. cyclische Polymere des Ethylenglykols der Formel (-OCH₂CH₂)p, wobei p eine Zahl z.B. von 4 bis 10 ist, wobei an den Ring auch eine oder mehrere Benzolringe ankondensiert sein können; Aza-und Thia-kronenether (Coronandamine und Coronand-sulfide); Glykole und teilweise veretherte Glykole, wie z.B. Ethylenglykol, Propylenglykol, Trimethylenglykol, Ethylenglykol-monomethyl-ether, Ethylenglykol-monoethyl-ether, Diethylenglykol-monoethyl-ether; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Benzophenon, Acetophenon; aliphatische Kohlenwasserstoffe, wie z.B. niedrig- und hochsiedende Petrolether; aromatische Kohlenwasserstoffe, wie z.B. Benzol, Toluol, o-, m- und p-Xylol; halogenierte aliphatische oder aromatische Kohlenwasserstoffe, wie z.B. Methylenchlorid, Chloroform, Tetrachlorkohlenstoff, Ethylen chlorid, Chlorbenzol, Dichlorbenzol; Nitrile, wie z.B. Acetonitril; Amide, wie z.B. Dimethylformamid, N-Methyl-pyrrolidon; Sulfoxide, wie z.B. Dimethyl-sulfoxid; Wasser. Auch Gemische verschiedener Lösungs- oder Dispergiermittel können verwendet werden.

Die Herstellung des Bis-esters der Formel II mit R³ a C₂H₅- ist z.B. in C. Grundmann, Chem. Ber. 97, (1964), 575-578, beschrieben. Andere Bis-ester der Formel II können analog hergestellt werden.

Die neuen Verbindungen der Formel I, bei denen R¹ die Gruppe -OR³, vorzugsweise -OC_{Z}H_{S}, bedeutet, und die demgemäß der Formel Ic entsprechen, 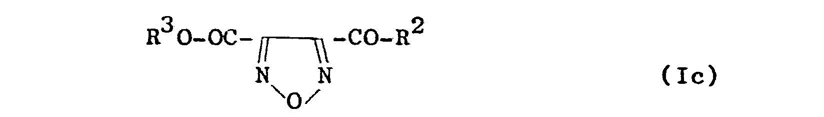 werden durch Umsetzung des Bis-esters der Formel II mit weniger als 2 mol, vorzugsweise mit 1 bis 1,1 mol, eines Amins der Formel R⁴NH₂ bzw. R⁵R⁶NH hergestellt. Die Reaktionsbedingungen bezüglich Temperatur und der bevorzugtenYerwendung eines Lösungs- oder Dispergiermittels und die in Betracht kommenden Lösungs- und Dispergiermittel sind dieselben, wie bei der Umsetzung des Bis-esters der Formel II mit 2 mol Amin. Die Monoamidbildung wird durch möglichst niedere Reaktionstemperaturen begünstigt.

Die neuen Verbindungen der Formel I, bei denen R¹ die Hydroxylgruppe bedeutet und die demgemäß der Formel Id entsprechen, 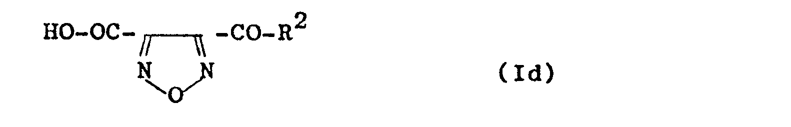 werden aus den Verbindungen der Formel Ic durch an sich bekannte hydrolytische Abspaltung des R³O-Restes hergestellt.

Diese hydrolytische Abspaltung wird mit ca. 10%iger Natriumhydroxydlösung bei 40 bis 80°C, vorzugsweise 45 bis 65°C, durchgeführt.

Neue Verbindungen der Formel I, bei denen R¹ die -NH-₂-Gruppe bedeutet und die demgemäß der Formel Ie entsprechen, 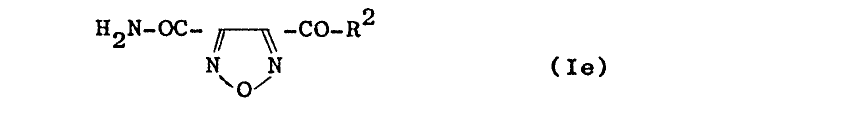 werden aus Verbindungen der Formel Ic durch Umsetzung mit Ammoniak hergestellt. Hierzu werden die Verbindungen der Formel Ic in einem der bereits genannten Lösungs- oder Dispergiermittel gelöst oder suspendiert und bei Temperaturen von 0°C bis 30°C Ammoniak in die Lösung oder Suspension eingeleitet.

Neue Verbindungen der Formel I, bei denen R¹ und R²verschiedene Aminreste bedeuten, werden aus Verbindungen der Formel Ic durch Umsetzung mit Aminen H₂NR⁴ oder HNR⁵R⁶ hergestellt. Die bei dieser Umsetzung angewandten Temperaturen und die vorzugsweise verwendeten Lösungs- oder Dispergiermittel entsprechen den Temperaturen und Lösungsmitteln, die bei der Herstellung der Verbindungen Ia und Ib durch Umsetzung des Bis-esters II mit mindestens 2 mol Amin zur Anwendung kommen. Bei der vorliegenden Umsetzung der Verbindung Ic werden solche Amine verwendet, deren Aminrest -NHR⁴ oder -NR⁵R⁶ verschieden von dem Rest R² ist. Bei der Herstellung von Verbindungen der Formel I, bei denen R1 und R² verschiedene Aminreste bedeuten, wird der Bis-ester II zunächst mit dem weniger reaktionsfreudigeren, selektiveren Amin zu der Verbindung Ic umgesetzt.

Sofern die Verbindungen der Formel Ic nur als Zwischenprodukte auftreten, brauchen sie nicht isoliert werden. Der Bis-ester der Formel II kann so zum Beispiel mit einem Mol eines weniger reaktionsfähigen selektiveren Amins NH₂R₄ oder HNR⁵R⁶ zu einer Verbindung der Formel Ic und sofort ohne Isolierung der Verbindung Ic mit dem zweiten Amin NH₂R'4 oder NHR'⁵R'⁶ umgesetzt werden. Die Reste R'⁴, R'⁵ und R'⁶ besitzen dabei eine der für R⁴ bzw. R⁵ und R⁶ angₑge- benen Bedeutungen, sind aber von R bzw. R⁵ und R⁶ verschieden.

Verbindungen der Formel I, bei denen R¹ und R² identisch sind, d.h. Verbindungen der Formeln Ia und Ib, lassen sich auch aus 1,2,5-Oxadiazol-2-oxid-3,4-bis-(carbonsdure-amiden) der Formel III 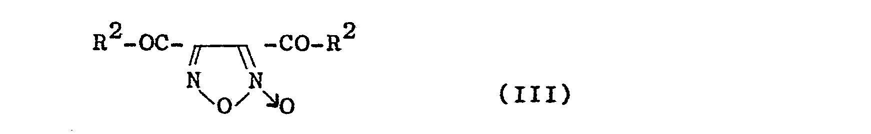 durch Reduktion herstellen. Als Reduktionsmittel eignen sich in Anlehnung an das von C. Grundmann loc. cit. beschriebene Verfahren vorzugsweise niedere Trialkylphosphite, d.h. solche, deren Alkylreste 1 bis 4 C-Atome besitzen, in einem inerten Lösungsmittel bei erhöhter Temperatur. Als Reduktionsmittel sind auch niedere Trialkyl-phosphine und Triphenylphosphin geeignet. Als Lösungsmittel sind z.B. aliphatische oder aromatische Kohlenwasserstoffe, wie z.B. nieder- und hochsiedender Petrolether, Benzol, Toluol oder ein Xylol, oder halogenierte Kohlenwasserstoffe, wie z.B. Chlorbenzol geeignet. Die Reaktionstemperaturen betragen 50 bis 150°C, vorzugsweise 100 bis 140°C. Je nach der erforderlichen Reaktionstemperatur muß ein Lösungsmittel mit einem entsprechenden Siedepunkt ausgewählt werden.

Die benötigten Ausgangsverbindungen der Formel III lassen sich aus Hydroxamoylchloriden der Formel IV durch HCl-Abspaltung und anschließende Dimerisierung herstellen:

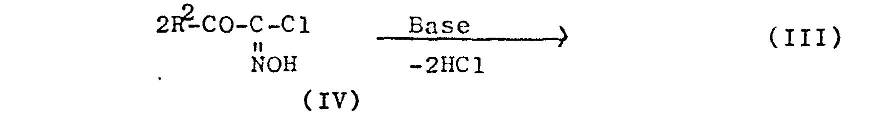

Die Reaktion wird in einem geeigneten Lösungs- oder Dispergiermittel, wie z.B. Dimethylformamid, Dimethylsulfoxid, N-Methylpyrrolidon, niederen Alkohol wie Methanol, Ethanol, vorzugsweise aber in Wasser durchgeführt. Auch Mischungen verschiedener Lösungs- oder Dispergiermittel, insbesondere homogene, aber auch heterogene Mischungen mit Wasser, wie z.B. Wasser/Methanol oder Wasser/Diethyläther, können verwendet werden. Die Reaktion wird im allgemeinen bei O bis 50°C, vorzugsweise bei 10 bis 25°C durchgeführt.

Nach dem Eintragen der Verbindung IV in das Lösungs- oder Dispergiermittel läuft die Reaktion häufig von selbst ab.

Sie kann jedoch durch Zusatz einer Base, die den abgespaltenen Chlorwasserstoff bindet, erheblich beschleunigt werden. Als derartige Base können z.B. benutzt werden:
primäre, sekundäre oder tertiäre organische Amine, wie z.B. Methylamin, Dimethylamin, Trimethylamin, Ethylamin, Diethylamin, Triethylamin; Pyridin; Alkalihydroxyde, wie z.B. Natrium- oder Kaliumhydroxyd; Alkalikarbonate und Alkalibikarbonate, wie z.B., Pottasche, Soda und Natriumbikarbonat; Alkaliacetate, wie z.B. Natriumacetat . Soda und Natriumbikarbonat sind bevorzugt. Die Base kann auch in Form einer Lösung (z.B. einer wäßrigen Lösung wie im Falle der niederen organischen Amine) oder einer Dispersion der Lösung oder Dispersion der Verbindung IV zugefügt werden. Wegen der eintretenden Reaktion ist eine allmähliche oder portionsweise Zugabe der Base und Umrühren des Reaktions-Gemisches zweckmäßig. Die Base wird vorzugsweise in molarer Menge (auf 2 mole der Verbindung IV 2 mole Base), gegebenenfalls mit einem bis zu 20%igen Überschuß zugesetzt. Nach beendeter Reaktion wird die gebildete Verbindung der Formel III abgetrennt.

Die Verbindungen der Formel IV können nach verschiedenen an sich bekannten Verfahren hergestellt werden, beispielsweise dadurch, daß zunächst Diketen V mit einem Amin VI zu einem Acetessigamid VII umgesetzt wird (vgl. z.B. US-PS 2 174 239):

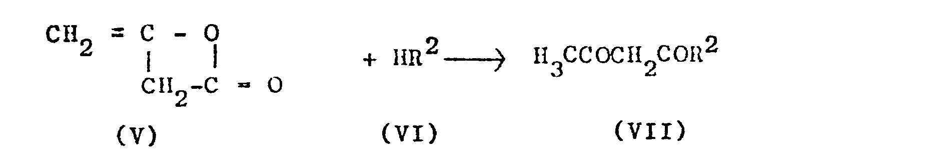

Die Umsetzung zwischen den Verbindungen V und VI wird in einem geeigneten Lösungsmittel, wie z.B. Wasser, oder einem Alkohol, normalerweise bei Temperaturen von 10 bis 50°C, vorzugsweise bei Raumtemperatur, durchgeführt.

Das Acetessigamid VII wird durch in situ erzeugte salpetrige Säure in einem geeigneten Lösungsmittel, wie Wasser, Eisessig oder einem Alkohol oximiert, wobei das Oxim VIII entsteht:

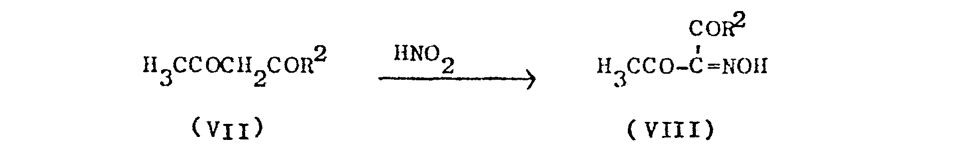

Diese Reaktion wird z.B. bei einem pH Wert von nicht weniger als 4,0 und bei Temperaturen von 10 bis 50°C, vorzugsweise bei bei Raumtemperatur, durchgeführt. Die salpetrige Säure wird am einfachsten aus Natrium-nitrit und Salzsäure erzeugt. Das Oxim VIII wird dann in einem geeigneten Lösungsmittel wie Wasser oder einem Alkohol z.B. bei Temperaturen von +10°C bis +70°C, vorzugsweise 30 bis 50°C, chloriert, wobei das Hydroxamoyl-chlorid IV entsteht.

Das Hydroxamoyl-chlorid IV kann aus dem Acetessigamid VII auch so hergestellt werden, daß man die Reihenfolge der Oximierung und Chlorierung vertauscht. Bei der Chlorierung entsteht dann zunächst aus dem Acetessigamid VII die Chlorverbindung IX: CH₃COCHClCOR², die dann durch Umsetzung mit salpetriger Säure in die Verbindung IV überführt wird.

Die Überführung von Acetessigamiden VII in Verbindungen der Formel IV nach den oben angegebenen Möglichkeiten ist z.B. beschrieben in der DE-AS 19 63 061. Ausgehend von Diketen V und dem Amin VI können die Ausgangsverbindungen III nach den vorstehend angegebenen Syntheseschritten ohne Isolierung der Zwischenprodukte in einem einzigen Reaktionsgefäß hergestellt werden.

Die Verbindungen I, die eine basische Seitenkette aufweisen, bilden mit anorganischen oder organischen Säuren Salze bzw. Säureadditionsverbindungen. Solche Säuren sind beispielsweise: Chlorwasserstoff-, Bromwasserstoff-, Phosphor-, Schwefel-, Oxal-, Milch-, Wein-, Essig-, Salicyl-, Benzoe-, Zitronen-, Ascorbin-, Adipin- und Naphthalindisulfonsäure. Die Säureadditionsverbindungen werden in bekannter Weise durch Vereinigen der Komponenten in einem geeigneten Lösungs- oder Dispergiermittel erhalten.

Die Verbindungen der Formel I und ihre pharmakologisch annehmbaren Säureadditionsverbindungen besitzen wertvolle pharmakologische Eigenschaften. Besonders ausgeprägt ist ihre Wirkung auf das Herz-Kreislauf-System. Sie senken in niedriger Dosierung den Blutdruck, vermindern den peripheren Widerstand und führen über eine Senkung des Pulmonalarteriendrucks bei unveränderter Herzfrequenz zu einer Verminderung der Herzarbeit. Die Verbindungen der Formel I und ihre pharmakologisch annehmbaren Säureadditionssalze können daher am Menschen als Heilmittel bei hohem Blutdruck und bei Angina pectoris sowie zur Prophylaxe des Angina-pectoris-Anfalls für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen. Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.I3. Lactose, Maisstärke oder Derivate davon,Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salzezur Veränderung des osmotischen Drucks, Uberzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat;Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon..

Die Dosierung kann innerhalb weiten Grenzen variieren und ist in jedem einzelnen Fall den individuellen Gegebenheiten anzupassen. Im allgemeinen ist bei oraler Verabreichung pro menschlichem Individuum eine Wirkstoff-Tagesdosis von etwa 0,4 bis 100 mg, vorzugsweise 1 bis 20 mg, angemessen. Auch bei anderen Applikationsformen liegt die Tagesdosis wegen der guten Resorption der Wirkstoffe in ähnlichen Mengenbereichen, d.h. im allgemeinen ebenfalls bei 0,4 bis 100 mg/ Mensch (pro kg Körpergewicht entspricht dies einer Tagesdosis von etwa 0,005 mg bis 1,4 mg, vorzugsweise von 0,013 mg bis 0,28 mg). Die Tagesdosis wird normalerweise in mehrere, z.B. 2 bis 4 Teilverabreichungen aufgeteilt. Die pharmazeutischen Zubereitungen enthalten von den Wirkstoffen pro Dosis im allgemeinen 0,1 bis 50 mg, vorzugsweise 0,5 bis 10 mg.

Die Untersuchungen über die antianginöse und antihypertensive Wirkung der Verbindungen der Formel I wurden an Bastardhunden beiderlei Geschlechts in Pentobarbital-Narkose (30 bis 40 mg/ kg i.v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit einem Ultrarotabsorptionsschreiber) betrug zwischen 4,5 und 5 Vol.%. Während des gesamten Versuchs erhielten die Tiere mit Pentobarbital-Narkose eine Dauerinfusion von Pentobarbital i.v. = 4 mg(in 6 ml)/kg/h, um eine konstante Narkosetiefe zu gewährleisten; die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Zur Bestimmung des mittleren Blutdrucks (=BDₘ) wurde der systolische und diastolische Blutdruck peripher in der Arteria femora1is über einen Statham-Druckaufnehmer gemessen. Ein über die Arteria carotis in den linken Ventrikel geschobener Millartip-Katheter lieferte das Signal für den linksventrikulären enddiaₛtolischen Druck (=LVEDP) und die Herzfrequenz (=HF). Mit einem zweiten, über die Venajugularis eingeschobenen Tip-Katheter wurde der mittlere Blutdruck (=PAP) in der Arteria pulmonalis erfaßt. Die erhaltenen Ergebnisse sind in der folgenden Tabelle angegeben:

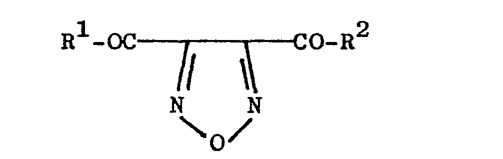

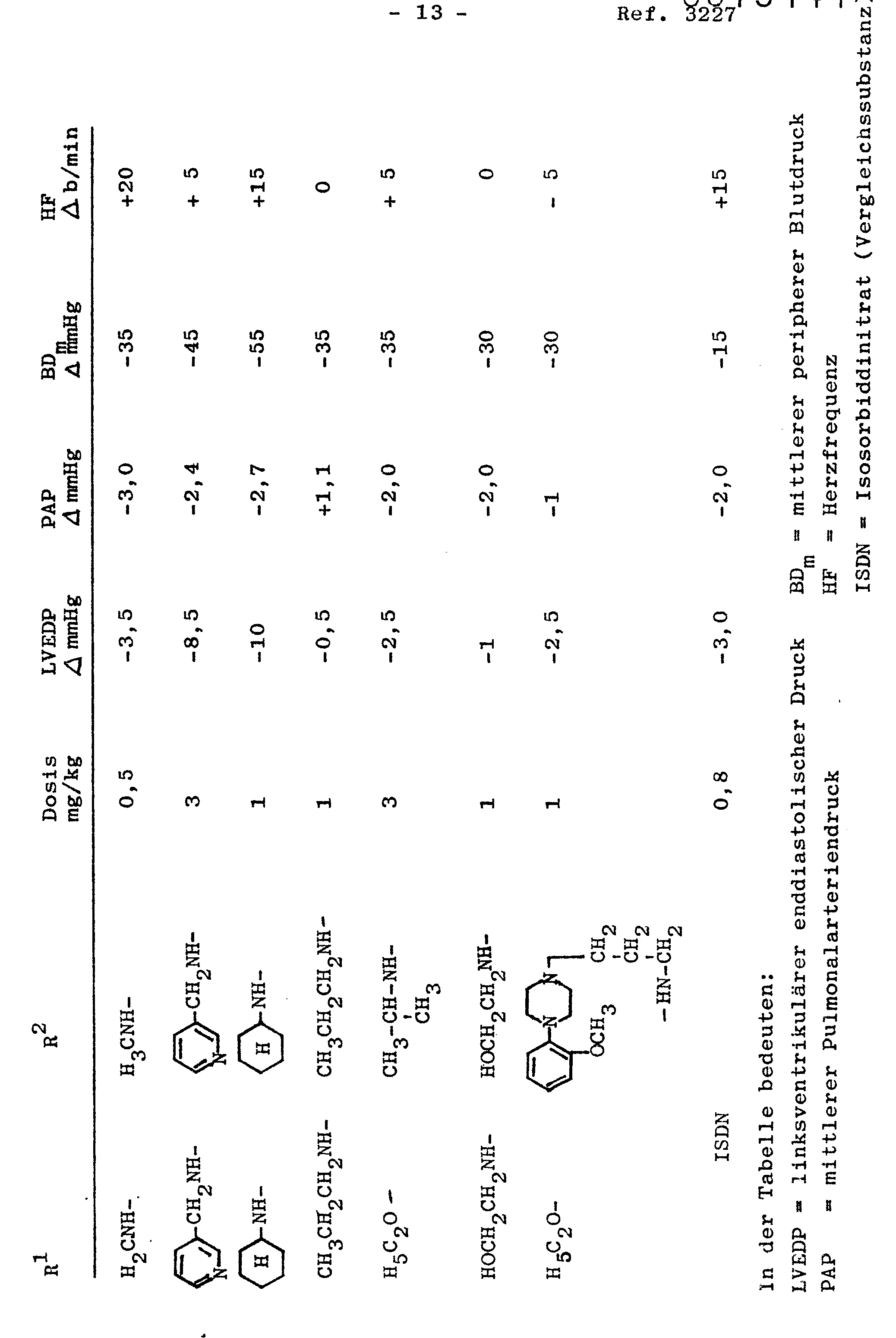

Die folgenden Beispiele dienen der weiteren Erläuterung der Erfindung, wobei Prozentangaben Gewichtsprozente bedeuten.

Zers. bedeutet Zersetzung.

### Beispiel 1

### l,2,5-0xadiazol-3,4-bis-(carbonsäuremethylamid)

8,0 g 1,2,5-Oxadiazol-2-oxid-3,4-bis-(carbonsäuremethylamid) und 7,0 g Triethylphosphit werden in 100 ml Toluol gelöst und 24 Stunden am Rückfluß gekocht. Die noch heiße Lösung wird filtriert und das Filtrat auf Raumtemperatur abgekühlt, wobei ein farbloser Niederschlag ausfällt, der aus 60 ml Isopropanol umkristallisiert wird:
5,3 g (72 % der Theorie), Fp = 161-163°C.

Analog diesem Beispiel werden hergestellt, wobei nach der Ausbeuteangabe das benutzte Reduktionsmittel, das Lösungsmittel und die Reaktionstemperaturen in °C angegeben sind. Bei den Reduktionsmitteln werden folgende Abkürzungen verwendet:
TBP-Tributylphosphit, TEP-Triethylphosphit, TMP-Trimethylphosphit, TIPP-Triisopropylphosphit, TPP-Triphenylphosphin:
   1,2,5-Oxadiazol-3,4-bis-(carbonsäureisopropylamid), Fp = 169-173°C (87 % der Theorie), TBP, Chlorbenzol, 130°C;
   1,2,5-Oxadiazol-3,4-bis-(carbonsäurepropylamid),Fp = 127-129°C (71 % der Theorie), TBP, Chlorbenzol, 130°C;
   1,2,5-Oxadiazol-3,4-bis-(carbonsäuremorpholid), Fp = 112 - 114°C (80 % der Theorie), TEP, Toluol, 110°C;
   1,2,5-Oxadiazol-3,4-bis-(carbonsäurepiperidid), Fp - 79 - 80°C (77 % der Theorie), TEP, Toluol, 110°C;
   1,2,5-Oxadiazol-3,4-bis-(carbonsäure-cyclohexylamid), Fp = 151 - 153°C (62 % der Theorie), TBP, o-Dichlorbenzol, 150°C;
   1,2,5-Oxadiazol-3,4-bis-(carbonsäuredimethylamid), Fp - 128 - 130°C (75 % der Theorie), TMP, Petrolether, 50°C;
   1,2,5-Oxadiazol-3,4-bis-(carbonsäurediethylamid), Fp - 116 - 118°C (78 % der Theorie), TMP, Toluol, 80°C;
   1,2,5-Oxadiazol-3,4-bis-(carbonsäurebutylamid), Fp = 112 - 114°C (80 % der Theorie), TEP, Toluol, 110°C;
   1,2,5-Oxadiazol-3,4-bis-(carbonsäurethylamid), Fp = 124 - 125°C (76 % der Theorie), TPP, Toluol, 110°C;
   1,2,5-Oxadiazol-3,4-bis-(carbonsäure-2-methoxiethylamid), Fp - 74 - 75°C (70 % der Theorie), TPP, Toluol, 110°C;
   1,2,5-Oxadiazol-3,4-bis-(carbonsäure-tert.-butylamid), Fp = 136 - 138°C (85 % der Theorie), TBP, Xylol, 140°C;
   1,2,5-Oxadiazol-3,4-bis-(carbonsäure-sec.-butylamid), Fp = 129 - 130°C (72 % der Theorie), TBP, Xylol, 140°C;
   1,2,5-Oxadiazol-3,4-bis-(carbonsäurecyclopentylamid), Fp = 163 - 165°C (73 % der Theorie), TIPP, Xylol, 120°C;
   1,2,5-4xadiazol-3,4-bis-(carbonsäurepyrrolidid), Fp - 87 - 88°C (71 % der Theorie), TEP, Toluol, 110°C;
   1,2,5-Oxadiazol-3,4-bis-(carbonsäure-3-methoxipropylamid, Öl (68 % der Theorie), TBP, Ligroin, 90°C.

Die benötige Ausgangsverbindung 1,2,5-Oxadiazol-2-oxid-3,4- bis(carbonsäure-methylamid) kann wie folgt hergestellt werden:
12,5 g Methylamin werden in 400 ml Wasser gelöst. 34 g Diketen werden bei Raumtemperatur (20°C) langsam zugetropft, wobei sich ein stabiler pH-Wert von 7 einstellt. In der erhaltenen Lösung werden dann 28 g Natriumnitrit gelöst. Konzentrierte Salzsäure (40 g) wird dann so zugetropft, daß der pH-Wert der Lösung nicht unter 4 absinkt. Nun wird 30 Min. lang nachgerührt und anschließend bei 20 - 40°C 30 g Chlor eingeleitet. Nach Abkühlen der Lösung wird auf -10°C abgekühlt und der Feststoff abgesaugt. Er wird in 100 ml Wasser aufgeschlämmt und bei 20°C vorsichtig portionsweise mit insgesammt 33,6 g Natriumbicarbonat versetzt. Die Lösung besitzt nun einen pH-Wert von 7,5 - 8. Nun wird auf O°C abgekühlt und der ausgefallene Feststoff abgesaugt und aus Isopropanol umkristallisiert. Farblose Kristalle vom Fp. 164 - 165°C.

In entsprechender Weise werden die folgenden 1,2,5-Oxadiazol-2-oxid-3,4-bis-carbonsäureamide hergestellt:
Bis-dimethylamid (Fp. 127 - 129°C), Bis-diethylamid (Öl), Bis-morpholid (Fp. 137 - 139°C), Bis-butylamid (Fp. 91 - 92°C), Bis-pyrrolidid (Fp. 106 - 108°C), Bis-ethylamid (Fp.116-117°C), Bis-isopropylamid (Fp.128-131°C), Bis-(2-methoxiethyl)amid (Fp. 94 - 95°C), Bisᵣpiperidid (Fp. 115-116°C), Bis-tert.-butylamid (Fp.166-167°C), Bis-cyclohexylamid (Fp. 137-138°C), Bis-sec.-butylamid (Öl), Bis-cyclopentylamid (Fp.144-146°C),

Bis-propylamid (Fp. 117 bis 118°C), Bis-(3-methoxipropyl)-amid (Öl). Auch die im Beispiel 2 angegebenen Verbindungen können nach dem Verfahren des Beispiels 1 hergestellt werden.

Die Strukturen der synthetisierten Verbindungen werden durch Elementaranalyse und durch die IR- und NMR-Spektren bestätigt.

### Beispiel 2:

### 1,2,5-Oxadiazol-3,4-bis-[carbonsäure-(3-pyridyl-methyl)-amid]

10,7 g 1,2,5-Oxadiazol-3,4-bis-(carbonsäureethylester) werden in 100 ml Ethanol vorgelegt. Die Lösung von 10,8 g(3-Pyridyl- methyl)-amin in 50 ml Ethanol wird bei Raumtemperatur langsam zugetropft und die Mischung 4 Stunden bei 20°C stehen gelassen. Nach dem Einengen wird aus Isopropanol (100 ml) umkristallisiert: 14,8 g farblose Nadeln (86 % der Theorie),Fp. = 108 bis 110°C.

Analog diesem Beispiel werden hergestellt, wobei nach der Ausbeuteangabe das benutzte Lösungsmittel oder Dispergiermittel und die Reaktionstemperatur in °C, sowie die Kohlenstoffzahl des bei der Umsetzung abgespaltenen Alkohols angegeben sind:
1,2,5-Oxadiazol-3,4-bis-(carbonsäure-tert.-butylamid), Fp - 136 - 138°C (71 % der Theorie), i-Propanol, 60°C, C-2;
1,2,5-Oxadiazol-3,4-bis-[carbonsäure-(2-{3,4-dimethoxiphenyl} -ethyl-amid], Fp = 105 bis 107°C (87 % der Theorie), Toluol, 110°C, C-2;
1,2,5-Oxadiazol-3,4-bis-(carbonsäure-methoxiethyl-amid), Fp - 74 bis 75°C (82 % der Theorie, Methanol 0°C, C-1;
1,2,5-Oxadiazol-3,4-bis-[carbonsäure-(4-methoxibenzyl)-amid], Fp = 116 bis 117⁰C (89 % der Theorie), Tetrahydrofuran, 50°C, C-1;
1,2,5-Oxadiazol-3,4-bis-(carbonsäure-ethanolamid), Fp - 93 bis 96°C (78 % der Theorie), Ethanol, 20°C, C-2;
1,2,5-Oxadiazol-3,4-bis-[carbonsäure-(2-diethyl-amino-ethyl)-amid], Öl, (68 % der Theorie), Dichlormethan, 30⁰C, C-2.

Auch die in Beispiel 1 angegebenen Verbindungen können nach dem Verfahren des Beispiels 2 hergestellt werden.

### Beispiel 3:

### 1,2,5-Oxadiazol-3-carbonsäureethylester-4-carbonsäure- isopropylamid

10,7 g 1,2,5-Oxadiazol-3,4-bis-carbonsäureethylester werden in 100 ml Ethanol vorgelegt und die Mischung auf O°C abgekühlt. Hierzu wird nun langsam die Lösung von 3,0 g Isopropylamin in 50 ml Ethanol getropft. Die Reaktionsmischung wird 2 Stunden bei 0°C gehalten und anschließend bei 20°C über Nacht stehen gelassen und dann am Rotationsverdampfer eingeengt. Die Aufarbeitung erfolgt über eine chromatographische Säulentrennung (Kieselgel, Methylenchlorid). Die zweite Fraktion wird gesammelt,und nach Verdampfen des Lösungsmittels wird der ölige Rückstand aus Toluol/Petrolether umkristallisiert. Fp - 70 - 72°C, Ausbeute: 2,5 g (23 % der Theorie).

Analog diesem Beispiel lassen sich herstellen:
1,2,5-Oxadiazol-3-carbonsäureethylester-4-carbonsäure-3-[N-2-methoxyphenyl-piperazino]-propylamid Fp (HCl-Salz) - 191 - 195⁰C (46 % der Theorie); [,2,5-Oxadiazol-3-carbon- säureethylester-4-carbonsäure7-N₋methylpiperazid, Fp (HCl-Salz) - 131 - 133⁰C (38 % der Theorie).

### Beispiel 4:

### 1,2,5-Oxadiazol-3-carbonsäureisopropylamid-4-carbonsaureamid

2,0 g 1,2,5-Oxadiazol-3-carbonsäureethylester-4-carbonsäure- isopropylamid werden in 50 ml Ethanol gelöst. In diese Lösung wird unter Kühlung Ammoniakgas bis zur Sättigung eingeleitet. Die Mischung wird 2 Stunden bei Raumtemperatur stehen gelassen, eingeengt und der zurückbleibende Feststoff aus Isopropanol umkristallisiert. 1,1 g,Fp = 173 - 176°C.

### Beispiel 5:

Zucker-überzogene Pillen können nach der folgenden Rezeptur hergestellt werden:

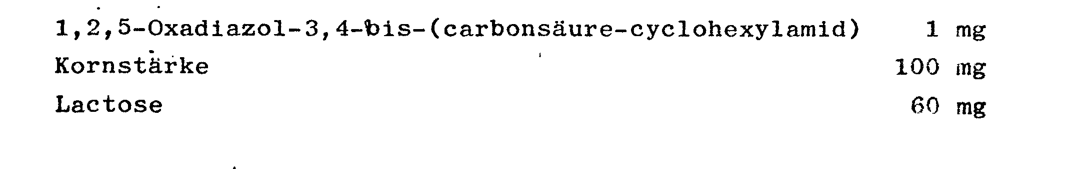

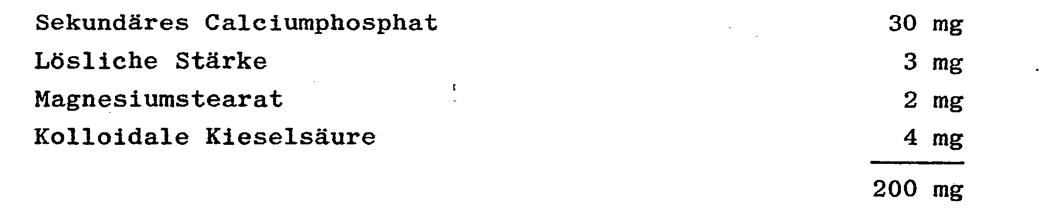

### Beispiel 6:

Tabletten können nach folgender Rezeptur hergestellt werden:

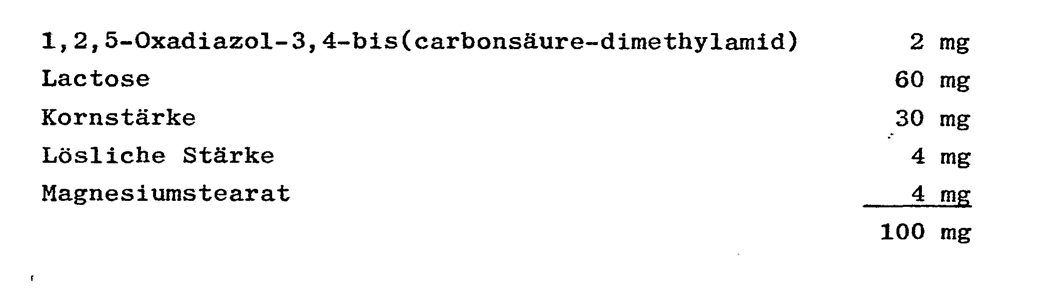

## Patentansprüche

1. 1,2,5-Oxadiazol-3,4-dicarbonsäurederivate der Formel I 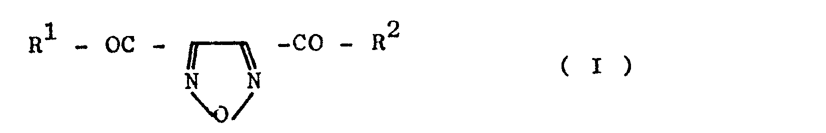 worin
R¹ die Gruppe -OH, -OR³, -NH₂, -NHR⁴ oder -NR⁵R⁶,
R² die Gruppe -NHR⁴ oder -NR⁵R⁶,
R³ eine Alkylgruppe mit 1 bis 4 C-Atomen,
R⁴ eine Alkylgruppe mit 1 bis 6 C-Atomen, eine Cycloalkylgruppe mit 5 oder 6 C-Atomen, eine Aralkylgruppe, die gegebenenfalls mit 1 oder 2 Methoxygruppen substituiert sein kann, oder eine Heteroarylalkylgruppe, eine -CH₂-CH₂-(CH₂)ₘ-OCH₃-Gruppe, worin m 0 oder 1 bedeutet, eine 2-Hydroxyethylgruppe oder eine basisch substituierte Alkylgruppe bedeuten und
_{R}⁵ und R⁶ unabhängig voneinander eine der Bedeutungen von
R⁴ besitzen oder zusammen mit dem Stickstoffatom, an das sie gebunden sind, einen gegebenenfalls substituierten, heterocyclischen Ring bilden, und ihre pharmakologisch annehmbaren Säureadditionsverbindungen.

2. 1,2,5-Oxadiazol-3,4-dicarbonsäurederivate nach Anspruch 1, dadurch gekennzeichnet, daß die für R⁴ stehende Aralkylgruppe eine Benzyl- oder Phenethylgruppe ist, die gegebenenfalls im Kern mit einem Methoxirest oder mit 2 Methoxiresten substituiert ist.

3. 1,2,5-Oxadiazol-3,4-dicarbonsäurederivate nach Anspruch 1, dadurch gekennzeichnet, daß die für R⁴ stehende Hetarylalkylgruppe eine Pyridylmethyl-gruppe ist.

4. 1,2,5-Oxadiazol-,3,4-dicarbonsäurederivate nach Anspruch 1, dadurch gekennzeichnet, daß die für R⁴ stehende basisch substituierte Alkylgruppe ein R⁷R⁸N-CH₂-(CH₂)ₘ-CH₂-Rest ist, worin m 0 oder 1 bedeutet und R⁷ und R⁸ Alkylreste mit 1 bis 4 C-Atomen oder zusammen mit dem Stickstoffatom, an das sie gebunden sind, gegebenenfalls substituierten heterocyclischen Rest bedeuten.

5. 1,2,5-Oxadiazol-3,4-bis(carbonsäure-dimethylamid).

6. 1,2,5-Oxadiazol-3,4-bis(carbonsäure-cyclohexylamid).

7. Verfahren zur Herstellung der Verbindungen nach einem oder mehreren der Ansprüche 1 bis 6, dadurch gekennzeichnet, daß ein 1,2,5-Oxadiazol-3,4-bis-carbonsäureester der Formel II 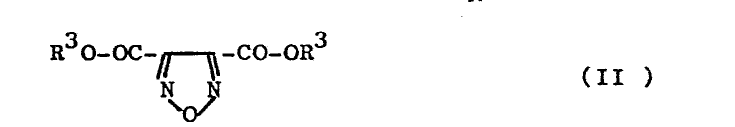 mit mindestens 2 Molen eines Amins R NH₂ oder R⁵R⁶NH umgesetzt wird, oder mit weniger als 2 Molen, insbesondere 1 bis 1,1 Molen, eines Amins R⁴NH₂ bzw. R⁵R⁶NH umgesetzt wird und gegebenenfalls anschließend mit NH₃ oder einem anderen Amin R⁴NH₂ oder R⁵R⁶NH umgesetzt oder zur Abspaltung der R³O-Gruppe hydrolysiert wird oder daß aus einem 1,2,5-0xadiazol-2-oxid-3,4-bis-(carbonsäure-amid) der Formel III 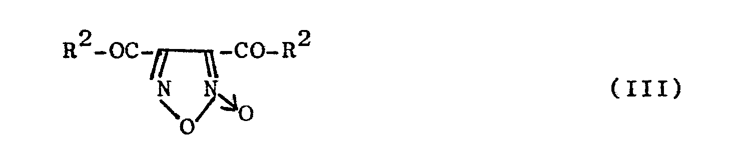 der Oxidsauerstoff durch Reduktion entfernt wird und die erhaltene Verbindung der Formel I gegebenenfalls in an sich bekannter Weise in eine Säureadditionsverbindung überführt wird.

8. Verwendung der 1,2,5-Oxadiazol-3,4-dicarbonsäurederivate nach einem oder mehreren der Ansprüche 1 bis 6 oder ihrer pharmakologisch annehmbaren Säureadditionsverbindungen als pharmakologische Wirkstoffe.

9. Verwendung der 1,2,5-0xadiazol-3,4-dicarbonsäurederivate nach einem oder mehreren der Ansprüche 1 bis 6 oder ihrer pharmakologisch annehmbaren Säureadditionsverbindungen als Heilmittel bei hohem Blutdruck oder bei Angina pectoris sowie zur Prophylaxe des Angina pectoris Anfalles.

10. Pharmazeutische Zubereitung, enthaltend eine wirksame Dosis eines 1,2,5-Oxadiazol-3,4-dicarbonsäurederivats nach einem oder mehreren der Ansprüche 1 bis 6 oder einer Säureadditionsverbindung davon neben pharmazeutisch zulässigen Träger- und Zusatzstoffen und gegebenenfalls noch anderen therapeutisch wirksamen Substanzen.

## Patentansprüche (Patentansprüche für folgende(n) Vertragsstaat(en) : AT)

1. Verfahren zur Herstellung von 1,2,5-Oxadiazol-3,4-dicarbonsäurederivaten der Formel I 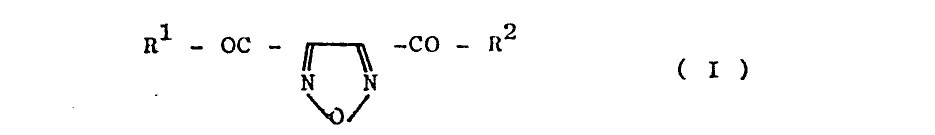 worin
R¹ die Gruppe -OH, -OR³, -NH₂, -NHR⁴ oder -NR⁵R⁶,
R² die Gruppe -NHR⁴ oder -NR⁵R⁶,
R³ eine Alkylgruppe mit 1 bis 4 C-Atomen,
R⁴ eine Alkylgruppe mit 1 bis 6 C-Atomen, eine Cycloalkylgruppe mit 5 oder 6 C-Atomen, eine Aralkylgruppe, die gegebenenfalls mit 1 oder 2 Methoxygruppen substituiert sein kann, oder eine Heteroarylalkylgruppe, eine -CH₂-CH₂-(CH₂)ₘ-OCH₃-Gruppe, worin m O oder 1 bedeutet, eine 2-IIydroxyethylgruppe oder eine basisch substituierte Alkylgruppe bedeuten und
R⁵ und R⁶ unabhängig voneinander eine der Bedeutungen von R⁴ besitzen oder zusammen mit dem Stickstoffatom, an das sie gebunden sind, einen gegebenenfalls substituierten heterocyclischen Ring bilden, und ihrer pharmakologisch annehmbaren Säureadditionsverbindungen, dadurch gekennzeichnet, daß ein 1,2,5-Oxadiazol-3,4-bis-carbonsäureester der Formel II 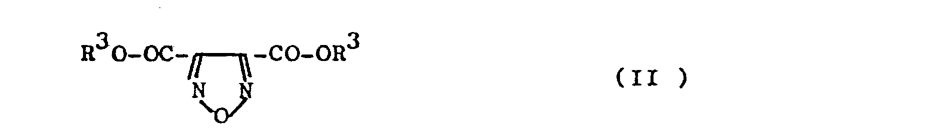 mit mindestens 2 Molen eines Amins R⁴NH₂ oder R⁵R⁶NH umgesetzt wird, oder mit weniger als 2 Molen, insbesondere 1 bis 1,1 Molen, eines Amins R⁴NH₂ bzw. R⁵R⁶NH umgesetzt wird und gegebenenfalls anschließend mit NH₃ oder einem anderen Amin R⁴NH₂ oder R⁵R⁶NH umgesetzt oder zur Abspaltung der R³O-Gruppe hydrolysiert wird oder daß aus einem 1,2,5-Oxadiazol-2-oxid-3,4-bis-(carbonsäure-amid) der Formel III 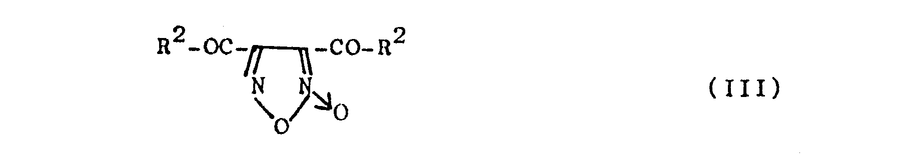 der Oxidsauerstoff durch Reduktion entfernt wird und die erhaltene Verbindung der Formel I gegebenenfalls in an sich bekannter Weise in eine Säureadditionsverbindung überführt wird.

2. Verfahren nach Anspruch 1, dadurch gekennzeichnet, daß die Verbindung der Formel II bei Temperaturen von -10 bis 120°C mit dem Amin umgesetzt wird.

3. Verfahren nach Anspruch 2, dadurch gekennzeichnet, daß die Umsetzung bei Temperaturen von 0 bis 120°C durchgeführt wird.

4. Verfahren nach Anspruch 2, dadurch gekennzeichnet, daß die Umsetzung bei Temperaturen von 0 bis 30°C durchgeführt wird.

5. Verfahren nach Anspruch 1, dadurch gekennzeichnet, daß die reduktive Entfernung des Oxidsauerstoffatoms aus der Verbindung der Formel III bei Temperaturen von 50 bis 150°C durchgeführt wird.

6. Verfahren nach Anspruch 5, dadurch gekennzeichnet, daß die reduktive Entfernung des Oxidsauerstoffatoms aus der Verbindung der Formel III bei Temperaturen von 100 bis 140°C durchgeführt wird.

7. Verfahren nach einem oder mehreren der Ansprüche 1 bis 4, dadurch gekennzeichnet, daß als Amin R⁴NH₂ Benzylamin, Phenethylamin, Pyridylmethylamin oder Cyclohexylamin eingesetzt wird.

8. Verfahren nach einem oder mehreren der Ansprüche 1 bis 4, dadurch gekennzeichnet, daß als Amin R⁵R⁶NH Dimethylamin eingesetzt wird.

9. Verfahren zur Herstellung eines gegen hohen Blutdruck oder Angina pectoris wirkenden Mittels, dadurch gekennzeichnet, daß ein 1,2,5-Oxadiazol-3,4-dicarbonsäurederivat der Formel I 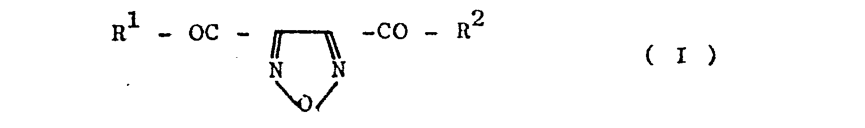 worin
R¹ die Gruppe -OH, -OR³, -NH₂, -NHR⁴ oder -NR⁵R⁶,
R² die Gruppe -NHR⁴ oder -NR⁵R⁶,
R3 eine Alkylgruppe mit 1 bis 4 C-Atomen,
R4 eine Alkylgruppe mit 1 bis 6 C-Atomen, eine Cycloalkylgruppe mit 5 oder 6 C-Atomen, eine Aralkylgruppe, die gegebenenfalls mit 1 oder 2 Methoxygruppen substituiert sein kann, oder eine Heteroarylalkylgruppe, eine -CH₂-CH₂-(CH₂)ₘ-OCH₃-Gruppe, worin m 0 oder 1 bedeutet, eine 2-Hydroxyethylgruppe oder eine basisch substituierte Alkylgruppe bedeuten und
R⁵ und R⁶ unabhängig voneinander eine der Bedeutungen von R⁴ besitzen oder zusammen mit dem Stickstoffatom, an das sie gebunden sind, einen gegebenenfalls substituierten, heterocyclischen Ring bilden, oder eine pharmakologisch annehmbare Säureadditionsverbindung hiervon, dadurch hergestellt wird, daß ein 1,2,5-Oxadiazol-3,4-bis-carbonsäureester der Formel II 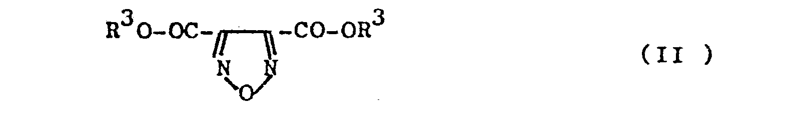 mit mindestens 2 Molen eines Amins R⁴NH₂ oder R⁵R⁶NH umgesetzt wird, oder mit weniger als 2 Molen, insbesondere 1 bis 1,1 Molen, eines Amins R⁴NH₂ bzw. R⁵R⁶NH umgesetzt wird und gegebenenfalls anschließend mit NH₃ oder einem anderen Amin R⁴NH₂ oder R⁵R⁶NH umgesetzt oder zur Abspaltung der R³O-Gruppe hydrolysiert wird oder daß aus einem 1,2,5-Oxadiazol-2-oxid-3,4-bis-(carbonsäure-amid) der Formel III 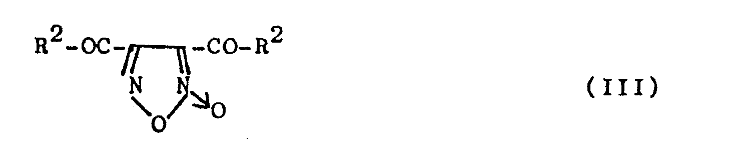 der Oxidsauerstoff durch Reduktion entfernt wird und die erhaltene Verbindung der Formel I gegebenenfalls in an sich bekannter Weise in eine pharmakologisch annehmbare Säureadditionsverbindung überführt wird und daß die so erhaltene Verbindung der Formel I oder ihre pharmakologisch annehmbare Säureadditionsverbindung mit einem geeigneten pharmazeutisch annehmbaren Trägerstoff gemischt wird.
